# EUROPEAN PATENT SPECIFICATION

(11) **EP 3 552 633 B1**
(45) Date of publication and mention of the grant of the patent: **06.11.2024**
(21) Application number: 18209799.8
(22) Date of filing: 03.12.2018
(51) Int. Cl.: A61L 9/04, A61L 9/12

(54) **IMPROVED AIR FRESHENER DISPENSER FOR CAR**
VERBESSERTER LUFTERFRISCHERSPENDER FÜR AUTO
DISPOSITIF DIFFUSEUR DE DÉSODORISANT AMÉLIORÉ POUR VOITURE

(30) Priority: 13.04.2018 ES 201830525
(43) Date of publication of application: 16.10.2019
(73) Proprietor: Rodriguez-Barbero Gonzalez, Ana Isabel, 28939 Madrid (ES)
(72) Inventor: Rodriguez-Barbero Gonzalez, Ana Isabel, 28939 Madrid (ES)
(74) Representative: Lorente Berges, Ana

(56) References cited:
- US-A- 4 341 348
- US-A1- 2012 104 113
- US-A1- 2014 048 617

## Description

### OBJECT OF THE INVENTION

The present invention belongs to the field of air freshener dispensers.

The object of the present invention is a new air freshener dispenser that doses of the air freshener better and maintains the fragrance longer.

### PRIOR ART OF THE INVENTION

Nowadays, a plurality of types of air freshener dispensers designed for providing a pleasant fragrance in any room are known.

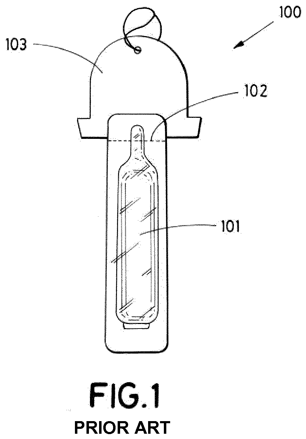

Fig. 1 shows a particular type of dispenser (100) formed by an air freshener flexible container (101) having an elongated shape with an end comprising a tearable area (102) for opening a hole for emitting the air freshener. This dispenser (100) may further comprise a flat part (103) made of an absorbent material and fixed to the container (101) at the end where the hole is located, such that when the tearable area (102) is broken, the flat part (103) is in a position adjacent said hole. Fig 1 shows a view of an air freshener (100) of this type where all decorative elements usually fitted to the product for sale are eliminated.

In order to use this prior art dispenser (100), the user first tears the tearable area (102) located at the upper end of the container (101) (represented by a broken line in Fig. 1), thus creating an outlet hole in the container (101). Then, the user presses the container (101) anywhere along its length, thus causing the air freshener to exit through the hole. The air freshener emitted through the hole impregnates the part (103) made of an absorbent material, and therefore the fragrance lasts longer in the surrounding environment.

A drawback of this type of dispensers is that possibly a portion of the air freshener exiting through the hole in the container is not absorbed by the part made of an absorbent material, and said portion of air freshener spills around the container and in the hands of the user. In this respect, account must be made that this type of air freshener usually is hanged in a vertical position, for example from a rear view mirror inside a car. Accordingly, it is not possible to ensure that all the air freshener exiting through the hole ends up in the part made of an absorbent material.

Another drawback of these dispensers is that controlling the quantity of air freshener emitted by the manual pressure of the user is not easy. That is, each user, or even the same user at different moments, presses the container with a difference strength, and thus the quantity of air freshener exiting through the hole changes in quantity and injection force.

Document US2012104113A1 discloses a vented dispensing device providing dispensable material in a closable container in concert with a corresponding accessory so that the container and the accessory can be commonly stored when not in use, and further provides a ventilated storage volume within the device that can facilitate drying and aerating the accessory.

Document US2014048617A1 discloses a dispensing system including a substrate and a mechanism for discharging a flowable medium through the substrate.

Document US4341348A discloses a fragrance dispensing device to provide a fragrance or scent by direct or indirect means comprising a container to discharge a quantity of a suitable fragrance, such as a perfume or deodorizer, and a cover assembly for the container to permit direct discharge in the form of a spray of the fragrance or scent and also to confine the spray discharge within the cover assembly where it is absorbed or adsorbed on a carrier member so that the fragrance or scent may be dissipated over a longer period of time.

### DESCRIPTION OF THE INVENTION

The present invention discloses a new air freshener dispenser for car that solves the aforementioned drawbacks. This new dispenser mainly comprises the following elements: an air freshener container; a dispensing nozzle; and an absorbent element. Next, each of these elements is disclosed in greater detail.

### a) Air freshener container

The air freshener container is, unlike the containers used in the prior art dispensers, rigid.

Regarding the shape, the container may have any shape as long as it has a closed cavity for receiving the air freshener. However, according to a preferred embodiment of the invention, the container may have an elongated shape, and more preferably an elongated prismatic shape. The prism may have any transversal cross-section, although preferably the cross-section is chosen between circular, elliptical, oval, and rectangular. Any polygonal shape could have rounded or chamfered edges.

Regarding the volume, the container may have any volume providing the dispenser with a reasonable duration. For example, the volume may be between approximately 2 ml and 10 ml.

### b) Dispensing nozzle

The dispensing nozzle is fitted to an upper end of the air freshener container such that a dose of air freshener is emitted every time the user presses said nozzle downwards. Additionally, the nozzle is longitudinally oriented with respect to the man direction of the container, that is, it is oriented upwards in the natural use position of the dispenser.

The dispensing nozzle is of the type user in the prior art for dispensing cologne or perfume. This type of nozzle is based on a spraying element connected with the inside of the container by means of a duct. When the user pushes down on the spraying element and overcomes the force of a spring, the spraying element moves downwards. When the user stops pushing, the spring urges the sprayer upwards, and said sprayer absorbs a predetermined dose of cologne or perfume that is emitted towards the surrounding environment.

Using this type of nozzles instead of the dispensing mechanism of the prior art dispensers allows for a much more precise dosing of the air freshener. Indeed, using the dispenser type shown in Fig. 1, each time the user presses the flexible container a different quantity of air freshener is emitted. On the contrary, using a nozzle of the present type, the quantity of air freshener emitted by each push is constant and known.

### c) Absorbing element

The absorbing element surrounds an outlet hole of the dispensing nozzle such that all the air freshener emitted through said nozzle impregnates said absorbing element.

This configuration is advantageous in that spilling the air freshener, as in prior art dispensers of the type shown in Fig. 1, is prevented. Air freshener spilling is not only unpleasant because the user's hands are stained, but they also may cause permanent marks on the dashboard of a car due to their corrosive characteristics.

In particular the absorbing element is formed by two parallel plates coupled to each other that enclose the outlet hole of the nozzle therebetween. The absorbing element thereby surrounds completely the outlet end of the dispensing nozzle such that all the air freshener impregnates said absorbing element.

This configuration is advantageous in that the flat plates are easy to produce and transport, while at the same time having a large evaporation surface for the air freshener.

In another preferred embodiment of the invention, an upper end of the absorbing element comprises a hole for hanging. This hole allows for hanging the dispenser of the invention, for example from the rear view mirror of a vehicle.

The dispenser disclosed solves the drawbacks of the prior art, since the possibility of air freshener spills is prevented, and it further allows for a better dosing of the air freshener.

### BRIEF DESCRIPTION OF THE FIGURES

Fig. 1 shows an air freshener dispenser according to the prior art.
Fig. 2 shows a perspective view of a first exemplary air freshener dispenser according to the present invention.
Fig. 3 shows a perspective side view of the exemplary air freshener dispenser of Fig. 2
Fig. 4 shows a view of a second exemplary air freshener dispenser according to the present invention.
Fig. 5 shows a view of the second exemplary air freshener dispenser of Fig. 5 having a decorative element attached thereto.

### PREFERRED EMBODIMENT OF THE INVENTION

An exemplary air freshener dispenser (1) according to the present invention is now disclosed in connection with the attached drawings.

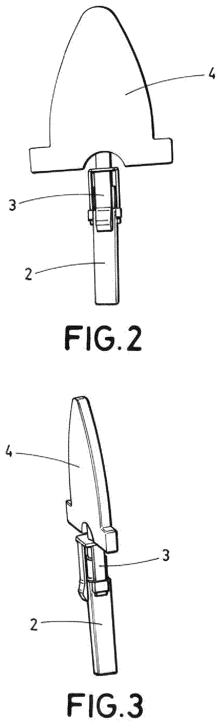

Figs. 2 and 3 show two perspective views of an air freshener dispenser (1) formed by a rigid container (2) connected at an end thereof to a dispensing nozzle (3), the nozzle having an end embedded in an absorbent element (4) formed by two essentially flat cellulose plates joined to each other. In this present example, the container (2) has the shape of a prism of essentially square cross-section having rounded edges. The nozzle (3) has a lateral protrusion designed for the user to push longitudinally downwards along the main direction of the container (2) for causing the emission of the air freshener. At and end of the nozzle (3) opposite to that where the container (2) is fitted, that is, at an upper end of the nozzle (3), the outlet hole (31) of the nozzle (3) through which emission of the air freshener takes place is located. This outlet hole (31) is completely embedded in the absorbing element (4). The absorbing element (4) is made of cellulose.

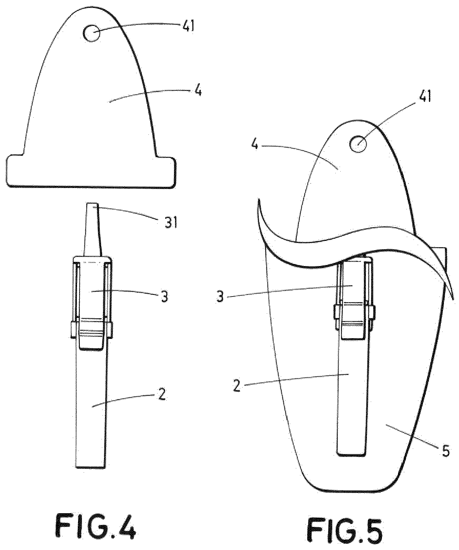

Figs. 4 and 5 show the aspect of the air freshener dispenser (1) when ready for sale to the public. Fig. 4 shows the unit formed by the container (2) and the nozzle (3) separated from the absorbing element (4). Fig. 5 shows the assembled unit having a decorative plate (5) for making it more pleasant for the public. In these figures, the hole (41) at the upper end of the absorbing element (4) for hanging the dispenser (1), for example from the rear view mirror of a vehicle, is shown.

## Claims

1. Air freshener dispenser (1) for car, comprising:
a rigid air freshener container (2);
a dispensing nozzle (3) coupled to an upper end of the air freshener container (2) such that it emits a dose of air freshener every time the user presses said nozzle (3) downwards; and
an absorbing element (4) surrounding an outlet orifice (31) of the dispensing nozzle (3),
**characterized in that**
the absorbing element (4) is formed by two parallel plates joined to each other and enclosing therebetween the outlet hole (31) of the nozzle (3), such that all the air freshener emitted by said nozzle (3) impregnates said absorbing element (4).

2. Air freshener dispenser (1) according to any of the previous claims, where an upper end of the absorbing element (4) comprises a hole (41) for hanging.

3. Air freshener dispenser (1) according to any of the previous claims, where the absorbing element (4) is made of cellulose.

4. Air freshener dispenser (1) according to any of the previous claims, where the container (2) has an elongated shape.

5. Air freshener dispenser (1) according to claim 4, where the container (2) having an elongated shape has a cross section chosen from the following list: circular, elliptical, oval, squared, rectangular.

6. Air freshener dispenser (1) according to any of the previous claims, where the container (2) has a volume of between 2 ml and 10 ml.

## Patentansprüche

1. Lufterfrischerspender (1) für das Auto, bestehend aus:
einem festen Lufterfrischerbehälter (2),
einer Spenderdüse (3), die an ein oberes Ende des Lufterfrischerbehälters (2) gekoppelt ist, so dass sie jedes Mal, wenn der Benutzer diese Düse (3) nach unten drückt, eine Dosis Lufterfrischer abgibt, und
einem absorbierendes Element (4), das eine Auslassöffnung (31) der Spenderdüse (3) umgibt,
**dadurch gekennzeichnet, dass**
das absorbierende Element (4) aus zwei parallelen, miteinander verbundenen Platten gebildet ist, die die Auslassöffnung (31) der Düse (3) so umschließen, dass der gesamte durch die Düse (3) abgegebene Lufterfrischer das absorbierende Element (4) imprägniert.

2. Lufterfrischerspender (1) gemäß einem der vorstehenden Ansprüche, bei dem ein oberes Ende des absorbierenden Elements (4) ein Loch (41) zum Aufhängen umfasst.

3. Lufterfrischerspender (1) gemäß einem der vorstehenden Ansprüche, dessen absorbierendes Element (4) aus Zellulose besteht.

4. Lufterfrischerspender (1) gemäß einem der vorstehenden Ansprüche, dessen Behälter (2) eine längliche Form aufweist.

5. Lufterfrischerspender (1) gemäß Anspruch 4, dessen länglich geformter Behälter (2) einen Querschnitt aufweist, der aus der folgenden Liste ausgewählt wird: kreisförmig, elliptisch, oval, quadratisch, rechteckig.

6. Lufterfrischerspender (1) gemäß einem der vorstehenden Ansprüche, dessen Behälter (2) ein Volumen zwischen 2 ml und 10 ml hat.

## Revendications

1. Distributeur de désodorisant (1) pour voiture, comprenant :
un récipient rigide de désodorisant (2) ;
une buse de distribution (3) couplée à une extrémité supérieure du récipient de désodorisant (2) de sorte qu'elle émet une dose de désodorisant chaque fois que l'utilisateur appuie sur ladite buse (3) vers le bas ; et
un élément absorbant (4) entourant un orifice de sortie (31) de la buse de distribution (3),
**caractérisé en ce que**
l'élément absorbant (4) est formé de deux plaques parallèles assemblées l'une à l'autre et entourant entre elles l'orifice de sortie (31) de la buse (3), de sorte que le désodorisant émis par ladite buse (3) imprègne ledit élément absorbant (4).

2. Distributeur de désodorisant (1) selon l'une quelconque des revendications précédentes, dans lequel une extrémité supérieure de l'élément absorbant (4) comprend un trou (41) pour la suspension.

3. Distributeur de désodorisant (1) selon l'une quelconque des revendications précédentes, dans lequel l'élément absorbant (4) est constitué de cellulose.

4. Distributeur de désodorisant (1) selon l'une quelconque des revendications précédentes, dans lequel le récipient (2) a une forme allongée.

5. Distributeur de désodorisant (1) selon la revendication 4, dans lequel le récipient (2) ayant une forme allongée a une section transversale choisie dans la liste suivante : circulaire, elliptique, ovale, carrée, rectangulaire.

6. Distributeur de désodorisant (1) selon l'une quelconque des revendications précédentes, dans lequel le récipient (2) a une capacité comprise entre 2 ml et 10 ml.
